# EUROPÄISCHE PATENTSCHRIFT

(11) **EP 2 252 272 B1**
(45) Veröffentlichungstag und Bekanntmachung des Hinweises auf die Patenterteilung: **29.03.2017**
(21) Anmeldenummer: 09721296.3
(22) Anmeldetag: 27.02.2009
(51) Int. Cl.: A61K 9/70, A61K 9/00, A61K 9/20

(54) **GINGIVAL-WAFER**
GINGIVAL WAFER
PLAQUETTE GINGIVALE

(30) Priorität: 15.03.2008 DE 102008014533
(43) Veröffentlichungstag der Anmeldung: 24.11.2010
(73) Patentinhaber: LTS LOHMANN Therapie-Systeme AG, 56626 Andernach (DE)
(72) Erfinder: ASMUSSEN, Bodo, 56170 Bendorf (DE); SIMON, Michael, 67105 Schifferstadt (DE)
(74) Vertreter: Schweitzer, Klaus
(86) Internationale Anmeldenummer: PCT/EP2009/001439
(87) Internationale Veröffentlichungsnummer: WO 2009/115178

(56) Entgegenhaltungen:
- EP-A- 0 641 560
- WO-A-95/09608
- US-A1- 2005 129 763
- US-A1- 2006 292 520
- P.H. LIST ET AL: "Hagers Handbuch der pharmazeutischen Praxis" 1971, SRINGER VERLAG , BERLIN-HEIDELBERG-NEW YORK , XP002557207 Seite 690 - Seite 691

## Beschreibung

Die vorliegende Erfindung betrifft streifenförmige Darreichungsformen zur Verabreichung eines Wirkstoffs über die Schleimhaut des Zahnfleisches.

Streifenförmige Produkte, die im Mund zur Anwendung kommen, werden im kosmetischen Bereich als Zahnpflegeprodukte angeboten. So sind zum Beispiel Zahnweiß-Streifen im Handel, die mit einer die Zähne bleichenden Zusammensetzung versehen sind und die auf die Zähne aufgelegt werden, um einen lang anhaltenden Kontakt zwischen den Zähnen und der bleichenden Zusammensetzung zu erreichen. Derartige Zahnweiß-Streifen werden beispielsweise in den Patentschriften US 5,879,691, US 6,045,811 und US 6,136,297 sowie der Offenlegungsschrift US 2006/0292520 A1 beschrieben.

Streifenförmige Produkte zur oralen Verabreichung von pharmazeutischen Wirkstoffen sind in Form von im Mundraum zerfallenden Darreichungsformen erhältlich. Diese streifenförmigen Darreichungsformen werden üblicherweise als "dünne, orale Streifen" oder "Wafer" bezeichnet. Bereits in Vermarktung befindliche Produkte dieser Art sind zum Beispiel die unter den Markenbezeichnungen Triaminic^{®}, Theraflu^{®}, Gas-X^{®} oder Benadryl^{®} vertriebenen Produkte. Bei diesen Produkten handelt es sich um Arzneizubereitungen, die für eine schnelle Zersetzung der Darreichungsform im Mund konzipiert sind. Durch ihre schnelle Zersetzung soll das Herunterschlucken des in ihr enthaltenen pharmazeutischen Wirkstoffs vereinfacht werden. Nach dem Herunterschlucken wird der pharmazeutische Wirkstoff im Gastrointestinaltrakt absorbiert. Daher ist der mit diesen kommerziell erhältlichen streifenförmigen Darreichungsformen verabreichte pharmazeutische Wirkstoff dem "First-Pass"-Metabolismus unterworfen.

Eine Möglichkeit, den "First-Pass"-Metabolismus zu umgehen, ist die Aufnahme von pharmazeutischen Wirkstoffen direkt über die Mundschleimhäute. Deren Durchblutung erfolgt über ein Netzwerk von Kapillaren, die einen direkten Zugang des Wirkstoffes in den systemischen Blutkreislauf ermöglichen. Dadurch kann der "First-Pass"-Metabolismus umgangen werden, indem ein Wirkstoff in das Kapillarnetzwerk der Mundschleimhaut diffundiert. Dazu muß ein vorzeitiges Herunterschlucken des Wirkstoffs verhindert und seine Absorption durch Schleimhäute in der Mundhöhle gefördert werden.

Die Mundhöhle wird von den Lippen, den Wangen, dem harten und dem weichen Gaumen, der Zunge und dem Mundboden begrenzt. Da die Schleimhäute des Mundraums leicht zugänglich sind, bietet sich dort ein besonders geeigneter Ort für eine transmukosale Wirkstoffverabreichung an. Zu den Mundschleimhäuten zählen die sublinguale Mukosa, die Wangenschleimhaut, die Mukosa des Zahnfleisches, die Schleimhäute der Gaumen sowie die Schleimhäute der Lippen. Der spezifische Applikationsort im Mundraum kann einen Einfluss auf die Bioverfügbarkeit eines Wirkstoffes haben. Die transmukosale Absorption von Wirkstoffen erfolgt in der Mundhöhle vorzugsweise über die nicht keratinisierten Schleimhäute, vor allem im Bereich der Wangen und unterhalb der Zunge. Wirkstoffe können aber auch über das keratinisierte Gewebe des Zahnfleisches absorbiert werden. Das Zahnfleisch als Applikationsort bietet drei große Vorteile: (A) eine sehr starke Durchblutung, so dass ein guter Zugang zum systemischen Blutkreislauf gegeben ist, (B) eine geringe mechanische Belastung einer dort angebrachten Arzneiform, z.B. bei Kaubewegungen, (C) eine geringe Umspülung mit Speichelflüssigkeit, so dass ein reduzierter Anteil an Arzneistoff verschluckt wird.

Ein wichtiger Einflussfaktor auf die Bioverfügbarkeit ist die Kontaktzeit zwischen absorptivem Epithelgewebe und dem Arzneistoff. Zwar ist es prinzipiell möglich, dass auch bei einer schnell zerfallenden Darreichungsform eine teilweise Absorption stattfindet, allerdings nur unter der Voraussetzung, dass der Wirkstoff günstige physikochemische Eigenschaften für eine transmukosale Absorption aufweist, z.B. geringes Molekulargewicht, lipophilen Charakter, etc. Für die Mehrzahl der Wirkstoffe, insbesondere Peptid-Wirkstoffe, deren transmukosale Verabreichung vorteilhaft wäre, ist eine längere Kontaktzeit zur absorbierenden Schleimhautoberfläche von entscheidender Bedeutung, damit der Wirkstoff in der für die therapeutische Wirkung erforderlichen Menge absorbiert werden kann.

Für eine Verabreichung von Wirkstoffen über die Mundschleimhäute sind neben Sprühlösungen vor allem verschiedene Tablettenformulierungen im Handel. Es gibt Sublingualtabletten, z.B. Nicorette^{®}-Microtabs, die Nikotin enthalten, oder Uprima^{®}-Tablets, die Apomorphin als Wirkstoff enthalten. Darüber hinaus gibt es Buccaltabletten, z.B. Fentora^{®}, die Fentanyl enthalten, oder Buccastem^{®}, die Prochlorperazin als Wirkstoff enthalten. Allerdings weisen derartige Tabletten einige Nachteile auf, zu denen (A) ein unangenehmes Mundgefühl gehört, weil sie ein Fremdkörpergefühl unter der Zunge verursachen, (B) Variabilitäten beim Absorptionsausmaß bestehen, und (C) die Gefahr des Verschluckens einer kompletten Tablette nicht ausgeschlossen werden kann.

Um diese Nachteile zu vermeiden oder zumindest abzuschwächen, wäre eine Arzneiform wünschenswert, mit der ein Wirkstoff zuverlässig über eine Schleimhaut im Mundraum verabreicht werden kann, wobei die Arzneiform an einer den Patienten nicht behindernden Stelle im Mund zur Anwendung kommt, an der sie auch über einen längeren Zeitraum hinweg getragen werden kann. Zudem wäre es von Vorteil, wenn die Arzneiform so gestaltet wäre, dass nur eine korrekte, für den Anwender selbsterklärende Positionierung möglich ist, um Fehler in der Anwendung zu vermeiden.

Diese Aufgabe wird mit der vorliegenden Erfindung in überraschend einfacher Weise gelöst, indem mit ihr eine streifenförmige Darreichungsform zur Anwendung im Mundraum bereitgestellt wird, insbesondere zum Bedecken eines Bereichs des äußeren Zahnfleisches. Die erfindungsgemäßen Darreichungsformen ermöglichen einen engen Kontakt mit der Schleimhaut des Zahnfleisches und eine lange Verweildauer auf dem absorbierenden Gewebe, wodurch die transmukosale Absorption eines Wirkstoffs durch die Schleimhaut des Zahnfleisches gefördert wird.

Gegenstand der vorliegenden Erfindung sind streifenförmige Darreichungsformen für die transmukosale Verabreichung eines Wirkstoffs über das Zahnfleisch, umfassend einen Materialstreifen, eine mukoadhäsive Schicht und mindestens einen Wirkstoff.

Die erfindungsgemäßen streifenförmigen Darreichungsformen weisen eine Kerbe in der Kante einer der beiden Längsseiten des Materialstreifens auf. Die Kerbe ist bei den erfindungsgemäßen Darreichungsformen üblicherweise in der Mitte der Längskante angeordnet. Die Kerbe kann aber auch zu einem der Enden des Materialstreifens hin angeordnet sein. Diese Kerbe ist eine Aussparung im Materialstreifen, durch die das obere Lippenbändchen (Frenulum labii superioris) oder das untere Lippenbändchen (Frenulum labii inferioris) führt, wenn die Darreichungsform angewendet wird. Die Lippenbändchen sind dünne, mit Mundschleimhaut bedeckte Bindegewebsfalten, die in den Mundvorhof hineinragen und sich in der Medianebene des Mundes von der Innenseite der Lippe zur Gingiva des Alveolarfortsatzes erstrecken. Das obere Lippenbändchen ist stärker ausgeprägt als das untere Lippenbändchen. Die Kerbe im Materialstreifen der erfindungsgemäßen Darreichungsform gewährleistet somit eine korrekte Positionierung der erfindungsgemäßen Darreichungsform auf dem Zahnfleisch. Dadurch wird die pharmakokinetische Reproduzierbarkeit, Anwendungssicherheit und Patientencompliance insbesondere für dauerhaft zu verabreichende Medikamente erheblich verbessert.

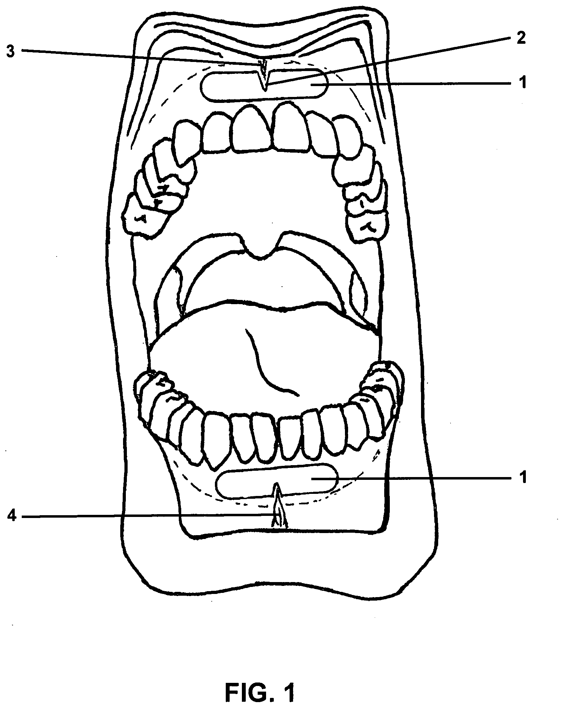

Figur 1 ist eine Zeichnung, welche die korrekte Platzierung erfindungsgemäßer Darreichungsformen auf dem oberen und unteren Zahnfleisch veranschaulicht.
Figuren 2A bis 2E stellen unterschiedliche Ausführungsformen der erfindungsgemäßen Darreichungsformen dar.
Figuren 3A bis 3E zeigen unterschiedliche Ausgestaltungsmöglichkeiten der Kerbe.

Die erfindungsgemäßen Darreichungsformen werden nachfolgend unter Bezugnahme auf die Figuren näher beschrieben. Dabei dienen die Figuren lediglich der beispielhaften Veranschaulichung der Erfindung, ohne ihren Umfang in irgendeiner Weise zu beschränken.

Die erfindungsgemäßen Darreichungsformen (1) beziehungsweise die Materialstreifen der streifenförmigen Darreichungsformen gemäß der vorliegenden Erfindung weisen vorzugsweise eine Höhe (h) von 0,3 cm bis 1,5 cm auf, besonders bevorzugt eine Höhe von 0,5 cm bis 1,0 cm.

Die erfindungsgemäßen Darreichungsformen beziehungsweise die Materialstreifen der streifenförmigen Darreichungsformen (1) gemäß der vorliegenden Erfindung weisen vorzugsweise eine Breite (b) von 1,0 cm bis 12,0 cm auf, besonders bevorzugt eine Breite von 2,0 cm bis 6,0 cm.

Die erfindungsgemäßen Materialstreifen der streifenförmigen Darreichungsformen weisen eine Dicke (d) von 10 µm bis 500 µm auf.

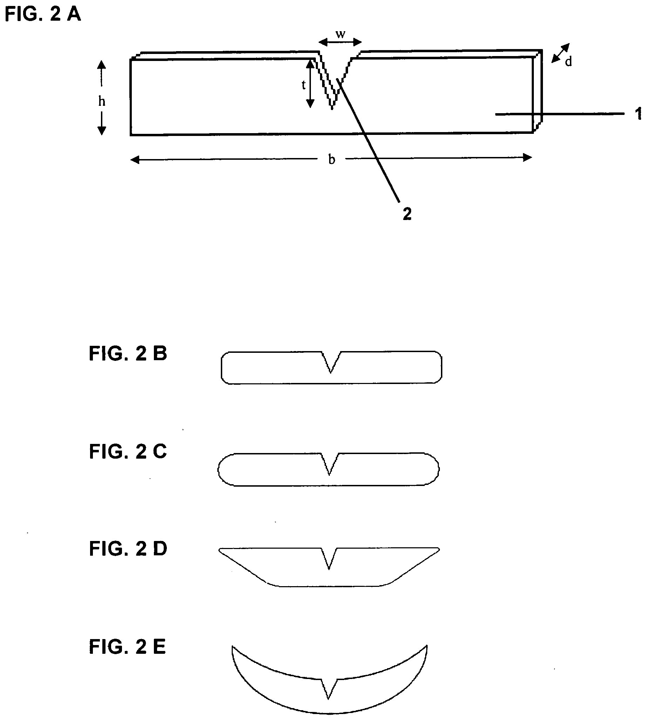

Die streifenförmigen Darreichungsformen (1) der vorliegenden Erfindung können unterschiedliche geometrische Formen aufweisen. Vorzugsweise weist die Darreichungsform eine bogenförmige Kontur, eine eckige Kontur, eine geradlinige Kontur oder eine Kontur auf, die sich aus der Kombination der genannten Konturen ergibt. Besonders bevorzugte geometrische Formen sind in den Figuren 2A bis 2E dargestellt.

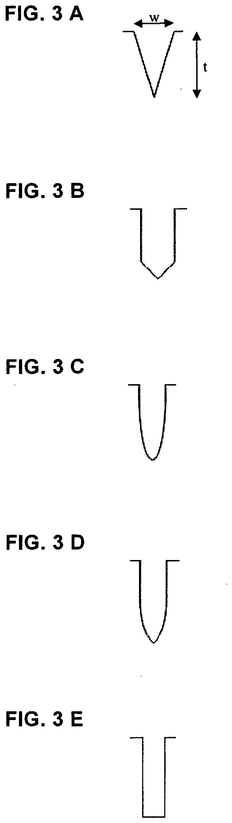

Die erfindungsgemäße Darreichungsform (1) weist eine Kerbe (2) an einer ihrer beiden Längskanten auf. Unter einer Kerbe wird eine Spalte, ein Einschnitt oder eine Aussparung verstanden, die in eine der beiden Längskanten des Materialstreifens gemacht wurde. Die sich an einer der beiden Längskanten des Materialstreifens befindende Kerbe (2) kann nahezu jede beliebige Form aufweisen. Besonders bevorzugt hat die Kerbe (2) eine bogenförmige Kontur, eine eckige Kontur, eine geradlinige Kontur oder eine Kontur, die sich aus der Kombination der genannten Konturen ergibt. Die Kerbe (2) kann beispielsweise keilförmig, rechteckig, spitz zulaufend, zumindest endständig polygonal, oder einem Oval oder Kreisausschnitt angenähert ausgestaltet sein. Besonders bevorzugte Ausgestaltungen der Kerbe (2) sind in den Figuren 3A bis 3E dargestellt.

Die Kerbe (2) weist eine Tiefe (t) von 2 mm bis 13 mm auf, bezogen auf die am weitesten eingeschnittene Stelle der Kerbe (2).

Die Tiefe der Kerbe (2) beträgt nicht weniger als die Hälfte der Höhe (h) des Materialstreifens.

Die Kerbe (2) weist vorzugsweise eine Weite (w) von 1 mm bis 10 mm auf, bezogen auf die größte Weite. Die größte Weite weist die Kerbe üblicherweise dort auf, wo ihr Rand auf die Längskante des Materialstreifens trifft.

Die Weite der Kerbe (2) beträgt in den bevorzugten Ausführungsformen die Hälfte bis ein Achthundertdreiunddreißigstel, vorzugsweise ein Zehntel bis ein Hundertstel, bezogen auf die Breite (b) des Materialstreifens.

Die Kerbe ist so dimensioniert und angeordnet, dass das obere Lippenbändchen (3) oder das untere Lippenbändchen (4) durch die Aussparung läuft, wenn die Darreichungsform korrekt auf dem Zahnfleisch platziert wird. Durch die Kerbe wird gewährleistet, dass die Darreichungsform nur in der vorgesehenen Weise und an der vorgesehenen Stelle auf dem Zahnfleisch angewendet werden kann, und auch bei wiederholter Anwendung diese Stelle immer die gleiche bleibt, wodurch auch die Einflussfaktoren auf die Arzneiform und folglich auf die Bioverfügbarkeit immer gleich bleiben.

Die streifenförmigen Darreichungsformen (1) gemäß der vorliegenden Erfindung können aus einem steifen Material oder einem flexiblen Material gefertigt sein. In Betracht kommen eine Vielzahl von Materialien, die für eine pharmazeutische und/oder kosmetische Verwendung durch Menschen und/oder Tiere geeignet sind, also keine unerwünschten Nebenwirkungen haben. Unerwünschte Nebenwirkungen wären beispielsweise toxische Wirkungen des Materials, das Hervorrufen von Reizungen oder das Auslösen allergischer Reaktionen. Geeignete Materialien können beispielsweise thermoplastische Polymere, duroplastische Polymere, Copolymerfilme, Papier, Wachse, Textilien (Gelege, Gewirke und Gewebe), Kreiden, Folien, Gele und Holzkomposite sowie Kombinationen der vorgenannten Materialien sein.

Spezifische Polymere, die als Streifenmaterial geeignet sind, können aus der Gruppe von Polymeren ausgewählt sein, die aus Celluloseethern, Methylacrylaten, Hydroxyalkylcellulosen wie Hydroxypropylmethylcellulose, Hydroxypropylcellulose, Hydroxyethylcellulose, Methylcellulose und Carboxymethylcellulose, Polysulfonen, Polyvinylpyrrolidonen, vernetzten Polyvinylpyrrolidonen, Polyvinylpyrrolidon-Vinylacetat-Copolymeren, Polyvinylalkoholen, Polyacrylsäuren, Polyacrylatpolymeren, vernetzten Polyacrylsäuren, Polyethylenoxiden, Polyethylenglycolen, Polyvinylalkylether-Maleimidsäure-Copolymeren und Carboxyvinylpolymeren besteht.

Geeignete Polymere können auch aus der Gruppe von Polymeren ausgewählt sein, die marine Kolloide, natürliche Gummen und Polysaccharide umfaßt. Zu diesen Polymeren gehören beispielsweise Natriumalginat, Carrageenan, Xanthangummi, Akaziengummi, Gummi arabicum, Guargummi, Pullulan, Agar, Chitin, Chitosan, Pektin, Karayagummi, Zein, Hordein, Gliadin, Johannisbrotkernmehl, Tragacanth und andere Polysaccharide, Stärken wie Maltodextrine, Amylose, Amylopektin, Maisstärke, Kartoffelstärke, Reisstärke, Tapiokastärke, Erbsenstärke, Süßkartoffelstärke, Gerstenstärke, Weizenstärke, wachsige Maisstärke, modifizierte Stärke, Dextrine, Lavan, Elsinan und Gluten; und Proteine wie Kollagen, Molkeeiweiß, Casein, Milcheiweiß, Sojaeiweiß, Gelatine, Wachse und Kolophonium, sowie synthetische Wachse und Bienenwachs.

Durch die Kombination von zwei oder mehreren der vorgenannten Polymere lassen sich die Eigenschaften des Materialstreifens wie Mukoadhäsivität, Flexibilität, Löslichkeitsverhalten, Quellverhalten und dergleichen entsprechend den Wünschen und Erfordernissen anpassen.

Der Materialstreifen oder die Schichten des Materialstreifens umfassen mindestens ein Polymer, das einen wesentlichen Bestandteil des Materialstreifens oder der Schicht(en) darstellt. Der Polymer-Anteil beträgt mindestens 5 Gew.-% und höchstens 90 Gew.-%, vorzugsweise 10 bis 70 Gew.-%, besonders bevorzugt 30 bis 60 Gew.-%, jeweils bezogen auf den Materialstreifen beziehungsweise die Schicht.

Der Materialstreifen oder einzelne Schichten des Materialstreifens können ferner Hilfs- oder Zusatzstoffe enthalten, um die chemischen oder physikalischen Eigenschaften zu beeinflussen, wie z. B. Flexibilität, mukoadhäsive Eigenschaften, Zerfallsfähigkeit, Quellfähigkeit und/oder Diffusionseigenschaften.

Als Hilfs- oder Zusatzstoffe kommen insbesondere Stoffe in Betracht, die aus der Antioxidationsmittel, Emulgatoren, Geliermittel, Geschmacksverstärker, Geschmackskorrigentien, Aromastoffe, Süßungsmittel, Stabilisatoren, pH-Regulatoren, Säuerungsmittel, Füllstoffe, Konservierungsmittel, Farbstoffe, Verdickungsmittel, Weichmacher und Feuchthaltemittel umfassenden Gruppe ausgewählt sind. Geeignete Hilfs- und Zusatzstoffe, die für pharmazeutische Anwendungen zugelassen sind, sind dem Fachmann bekannt.

Da viele Wirkstoffe, insbesondere peptidische Arzneistoffe, nur unzureichend über die Schleimhäute aufgenommen werden, ist der Zusatz von sogenannten Enhancern, das heißt Stoffen, die die Absorption ermöglichen und/oder beschleunigen, von großer Bedeutung.

Enhancer können aus den folgenden Stoffen bzw. Stoffgruppen ausgewählt werden: gesättigte oder ungesättigte Fettsäuren, Kohlenwasserstoffe, geradkettige oder verzweigtkettige Fettalkohole, Gallensalze und Gallensäurederivate, Cyclodextrinen, Dimethylsulfoxid, synthetische und nichtionische Emulgatoren, Phospholipide, Propylenglykol, Decanol, Dodecanol, 2-Octyldodecanol, Glycerin, Sorbitol, Mannitol und andere Zuckeralkohole, Isopropylidenglycerol, Transcutol (= Diethylenglycol-monoethylether), DEET (= N,N-Diethyl-m-Toluolamid), Solketal, Ethanol, 1,2-Propandiol oder andere Alkohole, Menthol und andere ätherische Öle oder Bestandteile ätherischer Öle, Laurinsäurediethanolamid, D-alpha-Tocopherol und Dexpanthenol, Chelatbildner wie EDTA (Ethylendiamintetraessigsäure); die vorstehende Aufzählung ist nicht abschließend.

Der Anteil dieser Hilfsstoffe kann vorzugsweise 0,5 bis 40 Gew.-% betragen, insbesondere 1 bis 30 Gew.-%, jeweils bezogen auf den Materialstreifen beziehungsweise die Schicht des Materialstreifens.

Der Materialstreifen kann aus Materialien hergestellt sein, die sich innerhalb einer bestimmten Zeit nach Anbringen der Darreichungsform am Zahnfleisch zersetzen. Die Darreichungsformen können den Wirkstoff in die Mundhöhle oder an die Schleimhaut des Zahnfleisches abgeben, bevor sie sich zersetzen, und/oder die Freisetzung des Wirkstoffs kann nach der Zersetzung der Darreichungsform erfolgen. Die Zersetzung der Darreichungsform kann auf beliebigem Wege erfolgen, beispielsweise durch mechanische, chemische und/oder physikalische Beanspruchung. So kann sich die Darreichungsform zersetzen, indem sie direkt oder nach einer chemischen Reaktion in Lösung geht. Bei einer mechanischen Beanspruchung kann es sich zum Beispiel um Schervorgänge oder Mahlprozesse handeln. Als physikalische Beanspruchung kann eine erhöhte Temperatur genannt werden. Die erfindungsgemäßen Darreichungsformen können beispielsweise in kleine Teile zerfallen, die optisch nicht voneinander unterscheidbar sind, oder es kann sich eine zusammenhängende Gelschicht bilden. Der Materialstreifen kann aber auch in wasserlösliche Bestandteile zerfallen, die während der Anwendung der Darreichungsform im Speichel in Lösung gehen.

Bei anderen Ausführungsformen der erfindungsgemäßen Darreichungsformen besteht der Materialstreifen aus mindestens einem in Wasser unlöslichen, jedoch zersetzungsfähigen Polymer, das in Wasser dispergiert werden kann. Das bedeutet, dass das Polymer in kleine Fragmente zerbricht. Das Polymer ist wasserunlöslich, aber quellfähig. In anderen Ausführungsformen, bei denen das Polymer während der Nutzung nicht zerbricht, kann das Polymer ein Wasser zurückweisendes Polymer oder ein wasserstabiles hydrophiles Polymer sein, wie bestimmte Cellulosearten, zum Beispiel Papier. In einigen Ausführungsformen kann der Zahnfleischstreifen eine Mischung von filmbildenden Materialien aufweisen.
Im Fall von wasserunlöslichen Materialstreifen oder bei wasserunlöslichen Schichten in mehrschichtigen Materialstreifen muss die nach Anwendung verbleibende(n) Schicht(en) nach einer vorgegebenen Zeit vom Zahnfleisch entfernt werden.

Die erfindungsgemäße Darreichungsform (1) umfasst einen mehrschichtigen Materialstreifen.

Zum Beispiel kann der Materialstreifen eine erste Schicht umfassen, die ein Polymer und/oder einen Klebstoff aufweist, eine zweite Schicht, die einen Wirkstoff oder eine funktionelle Zusammensetzung umfasst, und eine oder mehrere zusätzliche Schichten, die weitere oder zusätzliche Inhaltsstoffe bereitstellen oder der Darreichungsform besondere Eigenschaften verleihen.

Eine der äußeren Schichten ist mukoadhäsiv , um das Anhaften der Darreichungsform auf der Schleimhaut zu begünstigen und die Wirkstoffaufnahme über die Schleimhaut durch den direkten Kontakt zu vereinfachen.

Die Materialstreifen können markiert werden, um sicherzustellen, dass ein Patient die richtige Seite auf die Schleimhaut aufbringt, nämlich die Seite, über die der Wirkstoff an die Schleimhaut des Zahnfleisches abgegeben werden kann. Bei der Markierung kann es sich beispielsweise um eine Prägung in einer der Schichten handeln, um eine gefärbte Schicht oder um andere Markierungen.
Wasserunlösliche Schichten können als Schutzschichten verwendet werden, die verhindern, dass der Wirkstoff in die Mundhöhle freigesetzt wird, und eine Freisetzung zum Zahnfleischgewebe hin gewährleisten. Darüber hinaus kann der Wirkstoff in eine wasserunlösliche Schicht eingebettet sein, eine sogenannte Matrixschicht, aus der der Wirkstoff durch Porendiffusion über einen längeren Anwendungszeitraum hinweg freigesetzt wird. Die Darreichungsform muss nach erfolgter Wirkstoffabgabe entfernt werden, sofern sie aus nicht verdaubarem Material besteht.

Die erfindungsgemäßen streifenförmigen Darreichungsformen (1) weisen einen Wirkstoff auf. Bei dem Wirkstoff kann es sich um einen pharmazeutischen Wirkstoff, d. h. um einen Arzneistoff für therapeutische, prophylaktische oder diagnostische Zwecke, oder um einen kosmetischen Wirkstoff handeln.
Es gibt zahlreiche geeignete Wirkstoffe, deren Verabreichung mittels einer transmukosalen Darreichungsform vorteilhaft wäre, vor allem in der Gruppe der Analgetika, Antiarrhythmika, Antidementiva, Antidiarrhoika, Antiemetika, Antiepileptika, Antihypertonika, Antivertiginosa, Corticoide, Hormone, Kardiaka, Koronarmittel, Migränemittel, Neuroleptika, Psychopharmaka, Sedativa etc.

Für eine gingivale Absorption besonders bevorzugt sind folgende Wirkstoffe, bzw. deren Derivate und Salze: Alprazolam, Apomorphin, Acetylsalicylsäure, Buprenorphin, Captopril, Chlorpromazin, Codein, Cyanocobalamin, Dexamethason, Dextrometorphan, Diazepam, Diclofenac, Diltiazem, Domperidon, Ergotamin, Estradiol, Ethinylestradiol, Fentanyl, Isosorbiddinitrat, Levonorgestrel, Loperamid, Lorazepam, Methadon, Methylprednisolon, Methyltestosteron, Metoclopramid, Metronidazol, Miconazol, Morphin, Nalbuphin, Nifedipin, Nikotin, Nitroglycerin, Norelgestromin, Norethisteronacetat, Noscapin, Olanzapin, Omeprazol, Oxazepam, Oxybutynin, Pethidin, Prochlorperazin, Propranolol, Risperidon, Rotigotin, Testosteron, Timolol, Verapamil, Vitamin B₁₂.

Ganz besonders bevorzugt wäre die parenterale Applikation von peptidischen Arzneistoffen über die orale Schleimhaut, vor allem für Proteine mit einem Molekulargewicht < 10 kDa, z.B. Calcitonin, Desmopressin, GLP-1-Analoga wie Exenatide, Glucagon, GnRH-Analoga wie Buserelin, Insulin und -analoga, Leu-Enkephalin, Nafarelin, Oxytocin, Protirelin, Vasopressin und Somatostatin-Analoga wie Octreotid.

Diese Auflistung erhebt keinen Anspruch auf Vollständigkeit. Die beschriebene Darreichungsform kann prinzipiell mit jedem geeigneten Wirkstoff angewendet werden.

Der Materialstreifen der erfindungsgemäßen Darreichungsformen (1) fungiert als Träger für den Wirkstoff. Träger für den Wirkstoff zu sein, bedeutet jede Art der Beeinhaltung des Wirkstoffs. Beispielsweise kann der Wirkstoff in Form einer Dispersion, Emulsion oder Lösung sowie als Depot in Vertiefungen oder Taschen enthalten sein. Der Materialstreifen kann aber auch mit einer Dispersion, Emulsion oder Lösung eines Wirkstoffs imprägniert oder mit einer wirkstoffhaltigen Beschichtung versehen sein.

### Beispiel 1 (Referenzbeispiel):

Ein mukoadhäsiver Gingival-Wafer mit einschichtigem Aufbau wurde mit der folgenden Zusammensetzung (in Gew.-%) hergestellt:

| | |
|---|---|
| Lorazepam | 7,81% |
| Natrium Carboxymethylcellulose 7LF 25cP | 60,69% |
| Glycerin | 18,0% |
| Betacyclodextrin | 10,0% |
| Menthol | 2,5% |
| Natrium Saccharinat | 1,0% |

Die Form der einzelnen Wafer entsprach der in Fig. 2 C dargestellten Ausführungsform mit einer Breite (b) von 41 mm, einer Höhe (h) von 8 mm, einer Weite (w) der Kerbe von 5 mm, einer Tiefe (t) der Kerbe von 4 mm und einem Radius der seitlichen Rundungen von 4,2 mm. Das Flächengewicht (trocken) betrug 40 g/m², die Dicke (d) 50 µm.
Bei diesen Abmessungen betrug die Fläche des Wafers 3,2 cm² und enthielt eine Einzeldosis an Lorazepam von 1,0 mg.

### Beispiel 2:

Es wurde ein Gingival-Wafer mit zweischichtigem Aufbau hergestellt, der eine erste, wasserunlösliche Schicht als Schutzschicht und eine zweite, wirkstoffhaltige mukoadhäsive Schicht aufwies.

Die wirkstoffhaltige Schicht ist auf dem Zahnfleisch aufzubringen, während die wasserunlösliche Schicht ein Auflösen des Wirkstoffes im Mund und damit das Herunterschlucken des Wirkstoffes reduzieren soll. Zur Unterscheidung der beiden Schichten war die mukoadhäsive Schicht weiß und die Schutzschicht rot eingefärbt.

Die Schutzschicht bestand aus (in Gew.-%):

| | |
|---|---|
| Ethylcellulose N100 | 50,0% |
| Ethylcellulose N50 | 15,0% |
| Miglyol 812 | 34,0% |
| Eisenoxid rot E | 1,0% |

Die Schutzschicht wies ein Flächengewicht(trocken) von 45 g/m² und eine Dicke von 60 µm auf.

Die mukoadhäsive Schicht mit dem Wirkstoff Propranolol setzte sich zusammen aus (in Gew.-%):

| | |
|---|---|
| Propranolol HCl | 10,416% |
| Hydroxypropylmethylcellulose 50cPs | 73,084% |
| Polyethylenoxid WSR N-10 | 7,0% |
| Glycerin | 7,0% |
| Titandioxid | 2,5% |

Sie wies ein Flächengewicht von 150 g/m² auf und eine Dicke von 110 µm.

Die Dicke (d) des zweischichtigen Wafers betrug 170 µm. Die übrigen Abmessungen der einzelnen zweischichtigen Wafer entsprachen den Abmessungen, die in Beispiel 1 für eine einschichtige Ausführungsform angegeben sind. Bei einer Gesamtfläche von 3,2 cm² betrug die Einzeldosis von Propranolol 5,0 mg.

## Patentansprüche

1. Streifenförmige Darreichungsform für die transmukosale Verabreichung eines Wirkstoffs über das Zahnfleisch, umfassend einen Materialstreifen mit einer Dicke (d) von 10 bis 500 µm, der eine Kerbe (2) in der Kante einer der beiden Längsseiten des Materialstreifens aufweist, und mindestens einen Wirkstoff, wobei die Kerbe (2) eine Tiefe (t) von 2 mm bis 13 mm, bezogen auf die am weitesten eingeschnittene Stelle der Kerbe (2) aufweist und die Tiefe dieser Kerbe (2) nicht weniger als die Hälfte der Höhe (h) des Materialstreifens beträgt, **dadurch gekennzeichnet, dass** der Materialstreifen mehrschichtig ist, und die Darreichungsform eine äußere Schicht aufweist, die mukoadhäsiv ist.

2. Streifenförmige Darreichungsform nach Anspruch 1, **dadurch gekennzeichnet, dass** sie eine Höhe von 0,3 bis 1,5 cm, vorzugsweise von 0,5 bis 1,0 cm, aufweist.

3. Streifenförmige Darreichungsform nach Anspruch 1 oder 2, **dadurch gekennzeichnet, dass** sie eine Breite von 1,0 bis 12,0 cm, vorzugsweise von 2,0 bis 6,0 cm, aufweist.

4. Streifenförmige Darreichungsform nach einem der vorangehenden Ansprüche, **dadurch gekennzeichnet, dass** sie eine Dicke von 20 bis 300 µm, aufweist.

5. Streifenförmige Darreichungsform nach einem der vorangehenden Ansprüche, **dadurch gekennzeichnet, dass** die Kerbe keilförmig, rechteckig, spitz zulaufend oder zumindest endständig polygonal, oder einem Oval oder Kreisausschnitt angenähert, ist.

6. Streifenförmige Darreichungsform nach einem der vorangehenden Ansprüche, **dadurch gekennzeichnet, dass** die Kerbe eine Breite von 1 bis 10 mm, bezogen auf die breiteste Stelle, aufweist.

7. Streifenförmige Darreichungsform nach einem der vorangehenden Ansprüche, **dadurch gekennzeichnet, dass** der Materialstreifen aus einem flexiblen Material besteht, das sich an die Form des Zahnfleisches eines Anwenders anpasst.

8. Streifenförmige Darreichungsform nach einem der vorangehenden Ansprüche, **dadurch gekennzeichnet, dass** der Wirkstoff ein kosmetischer oder pharmazeutischer Wirkstoff ist.

9. Streifenförmige Darreichungsform nach einem der vorangehenden Ansprüche, **dadurch gekennzeichnet, dass** der Materialstreifen markiert ist, wobei es sich bei der Markierung vorzugsweise um eine Prägung in einer der Schichten oder um eine gefärbte Schicht handelt.

10. Streifenförmige Darreichungsform nach einem der vorangehenden Ansprüche, **dadurch gekennzeichnet, dass** sie eine wasserunlösliche Schicht aufweist.

11. Streifenförmige Darreichungsform nach einem der vorangehenden Ansprüche zur transmukosalen Verabreichung eines Arzneistoffs über die Schleimhaut des Zahnfleisches, **dadurch gekennzeichnet, dass** die Darreichungsform bei der Anwendung in der Weise auf dem Zahnfleisch positioniert wird, dass das obere Lippenbändchen (3) oder das untere Lippenbändchen (4) durch die Kerbe (2) läuft.

## Claims

1. Strip-shaped administration form for transmucosal administration of an active substance via the gums, comprising a strip of material which has a thickness (d) of 10 to 500 µm and has a notch (2) in the edge of one of the two longitudinal sides of the strip of material, and at least one active substance, wherein the notch (2) has a depth (t) of 2 mm to 13 mm, relative to the point where the notch (2) is cut deepest, and the depth of this notch (2) is not less than half the height (h) of the strip of material, **characterized in that** the strip of material is multi-layered, and the administration form has an outer layer which is mucoadhesive.

2. Strip-shaped administration form according to Claim 1, **characterized in that** it has a height of 0.3 to 1.5 cm, preferably of 0.5 to 1.0 cm.

3. Strip-shaped administration form according to Claim 1 or 2, **characterized in that** it has a width of 1.0 to 12.0 cm, preferably of 2.0 to 6.0 cm.

4. Strip-shaped administration form according to one of the preceding claims, **characterized in that** it has a thickness of 20 to 300 µm.

5. Strip-shaped administration form according to one of the preceding claims, **characterized in that** the notch is wedge-shaped, rectangular, tapered to a point, or at least terminally polygonal, or approximates to an oval or to a circular cut-out.

6. Strip-shaped administration form according to one of the preceding claims, **characterized in that** the notch has a width of 1 to 10 mm, relative to the widest point.

7. Strip-shaped administration form according to one of the preceding claims, **characterized in that** the strip of material is made of a flexible material that conforms to the shape of the gums of a user.

8. Strip-shaped administration form according to one of the preceding claims, **characterized in that** the active substance is a cosmetic or pharmaceutical active substance.

9. Strip-shaped administration form according to one of the preceding claims, **characterized in that** the strip of material is marked, the marking preferably being an embossing in one of the layers or being a coloured layer.

10. Strip-shaped administration form according to one of the preceding claims, **characterized in that** it has a water-insoluble layer.

11. Strip-shaped administration form according to one of the preceding claims for transmucosal administration of a medicament via the mucous membrane of the gums, **characterized in that** the administration form, during use, is positioned on the gums in such a way that the frenulum (3) of the upper lip or the frenulum (4) of the lower lip extends through the notch (2).

## Revendications

1. Forme galénique en bande pour l'administration transmucosale d'une substance active sur la gencive, comprenant une bande de matériau d'une épaisseur (d) de 10 à 500 µm, qui présente une entaille (2) dans l'arête d'un des deux longs côtés de la bande de matériau, et au moins une substance active, dans laquelle l'entaille (2) présente une profondeur (t) de 2 mm à 13 mm, par rapport à l'endroit le plus entaillé de l'entaille (2) et la profondeur de cette entaille (2) n'est pas inférieure à la moitié de la hauteur (h) de la bande de matériau, **caractérisée en ce que** la bande de matériau est multicouche, et la forme galénique présente une couche extérieure, qui est mucoadhésive.

2. Forme galénique en bande selon la revendication 1, **caractérisée en ce qu'**elle présente une hauteur de 0,3 à 1,5 cm, de préférence de 0,5 à 1,0 cm.

3. Forme galénique en bande selon la revendication 1 ou 2, **caractérisée en ce qu'**elle présente une largeur de 1,0 à 12,0 cm, de préférence de 2,0 à 6,0 cm.

4. Forme galénique en bande selon l'une quelconque des revendications précédentes, **caractérisée en ce qu'**elle présente une épaisseur de 20 à 300 µm.

5. Forme galénique en bande selon l'une quelconque des revendications précédentes, **caractérisée en ce que** l'entaille est en forme de coin, rectangulaire, pointue ou au moins d'extrémité polygonale, ou proche d'un ovale ou d'un secteur circulaire.

6. Forme galénique en bande selon l'une quelconque des revendications précédentes, **caractérisée en ce que** l'entaille présente une épaisseur de 1 à 10 mm, par rapport à l'endroit le plus large.

7. Forme galénique en bande selon l'une quelconque des revendications précédentes, **caractérisée en ce que** la bande de matériau est constituée d'un matériau souple, qui s'adapte à la forme de la gencive d'un utilisateur.

8. Forme galénique en bande selon l'une quelconque des revendications précédentes, **caractérisée en ce que** la substance active est une substance active cosmétique ou pharmaceutique.

9. Forme galénique en bande selon l'une quelconque des revendications précédentes, **caractérisée en ce que** la bande de matériau est marquée, dans laquelle le marquage est de préférence une empreinte dans une des couches ou une couche colorée.

10. Forme galénique en bande selon l'une quelconque des revendications précédentes, **caractérisée en ce qu'**elle présente une couche insoluble dans l'eau.

11. Forme galénique en bande selon l'une quelconque des revendications précédentes pour l'administration transmucosale d'une substance active sur la muqueuse de la gencive, **caractérisée en ce que** la forme galénique est positionnée sur la gencive lors de l'application de façon à ce que le frein labial supérieur (3) ou le frein labial inférieur (4) passe par l'entaille (2).
